# EUROPEAN PATENT SPECIFICATION

(11) **EP 2 943 244 B1**
(45) Date of publication and mention of the grant of the patent: **03.01.2018**
(21) Application number: 14702360.0
(22) Date of filing: 10.01.2014
(51) Int. Cl.: A61M 25/09

(54) **CORONARY GUIDEWIRE**
KORONARER FÜHRUNGSDRAHT
FIL-GUIDE CORONAIRE

(30) Priority: 10.01.2013 US 201361751029 P
(43) Date of publication of application: 18.11.2015
(73) Proprietor: THE CLEVELAND CLINIC FOUNDATION, Cleveland, OH 44195 (US); Yokowo Co., Ltd, Tokyo 114-8515 (JP)
(72) Inventor: WHITLOW, Pat, Cleveland, Ohio 44195 (US); OOKA, Naoki, Tokyo 114-8515 (JP); SAKAI, Koichi, Tokyo 114-8515 (JP)
(74) Representative: Wilson Gunn
(86) International application number: PCT/US2014/010978
(87) International publication number: WO 2014/110326

(56) References cited:
- EP-A1- 0 739 641
- EP-A1- 1 498 152
- EP-A1- 1 803 483
- EP-A1- 2 481 440
- EP-A1- 2 505 226
- EP-A2- 2 505 225
- WO-A1-2012/041905
- WO-A2-96/25969
- US-A- 5 551 444
- US-A1- 2002 082 523
- US-A1- 2005 054 950
- US-A1- 2006 264 784
- US-A1- 2007 185 415
- US-A1- 2007 249 964

## Description

### Related Applications

This application claims the benefit of U.S. Provisional Application Serial No. 61/751,029, filed on January 10, 2013.

### Technical Field

The present invention relates generally to a guidewire and, more particularly, to a coronary guidewire with a distal end portion adapted to advance through occlusions in a vessel.

### Background of the Invention

The present invention relates in general to the field of medical devices and, in particular, to devices for use in interventional and diagnostic access, manipulation within, and negotiation of, the vascular system.

The vascular field of medicine relates to the diagnosis, management and treatment of diseases affecting the arteries and veins. Even when healthy, the anatomy of these vessels is complex, with numerous divisions leading into progressively smaller branches. Development of disease within these vessels often complicates matters by altering their caliber, flexibility, and direction. The interior, or lumen, of a blood vessel may develop constrictions, known as stenoses, and at times may even be obstructed, as a result of the development of atherosclerotic plaques or by the occurrence of tears or lacerations in the vessel wall, known as dissections. These obstructions may complicate the vascular anatomy by leading to the formation of new collateral pathways that establish new routes around the obstructions in order to provide blood flow down-stream from the blockage.

In order to diagnose and treat vascular diseases, a physician may in many instances perform a diagnostic or interventional angiogram. An angiogram is a specialized form of X-ray imaging, requiring physical access into a vessel with some form of cover, needle or guide in order to allow a contrast dye to be injected into the vasculature while X-rays are transmitted through the tissue to obtain an image. The contrast dye illuminates the interior of the vessels and allows the physician to observe the anatomy, as well as any narrowings, abnormalities, or blockages within the vessels. At times, more selective angiograms are used to delineate a particular area of concern or disease with greater clarity. Access to these more selective areas often requires the insertion of guidewires and guide catheters into the vessels.

Vascular guidewires and guide catheters can be visualized from outside the body, even as they are manipulated through the body's vascular system, through the use of continuous low-dose fluoroscopy. The negotiation of the complex vascular anatomy, even when healthy, can be difficult, time consuming and frustrating. When narrowed or obstructed by disease, the vessels are even more difficult-and sometimes impossible-to negotiate.

Attempts to address and overcome the difficulty of negotiating vascular anatomy have led to various devices, primarily guidewires and guide catheters, for assisting physicians. The devices vary in shape, diameter and length. In order to negotiate the smaller blood vessels as well as to provide some standardization within the industry, for example, many catheterization systems are sized to cooperate with guidewire diameters of 0.035" (0.89mm) or less (0.018" and 0.014" (0.045 and 0.035mm) being the next most common sizes).

### Summary of the Invention

The invention relates to a coronary guidewire (as claimed in claim 1) that includes a flexible elongated core and a coil comprising a plurality of helical coil turns wound around a distal end portion of the core. The coil has a proximal end and an opposite distal end. A weld joint connects the distal end of the coil to the core and defines a distal tip of the guidewire. The weld joint can be a tungsten inert gas welded joint. The guidewire may include a solder joint that connects the proximal end of the coil to the core. A second solder joint may connect the coil to the core at a location between the welded distal end of the coil and the soldered proximal end of the coil.

The coil may include a proximal portion including the proximal end of the coil and a distal portion including the distal end of the coil. The proximal portion of the coil can be constructed of stainless steel, and the distal portion of the coil can be constructed of a platinum alloy. The distal end portion of the core can include a bend adjacent the distal tip. The bend in the core can have a bend radius in the range of 0.2mm-0.6mm. The coil may follow the bend such that adjacent coil turns along the bend are spaced from each other and can be moved toward each other to permit the bend in the core to straighten.

### Brief Description of the Drawings

For a better understanding of the invention, reference may be made to the accompanying drawings, in which:
Fig. 1 is a plan view illustrating a guidewire, according to the invention;
Fig. 2 is a plan view illustrating a portion of the guidewire of Fig. 1;
Fig. 3 is a plan view illustrating the guidewire of Fig. 1 in a different condition;
Fig. 4 is a magnified view of a portion of the guidewire of Fig. 3; and
Figs. 5A-5E are schematic views illustrating the operation of the guidewire of Figs. 1-4.

### Description of Embodiments

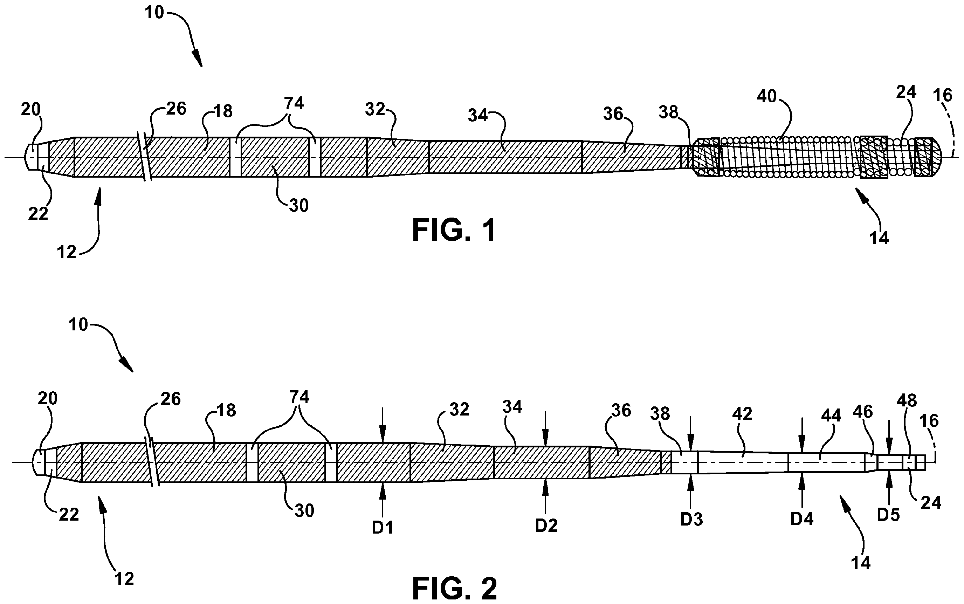

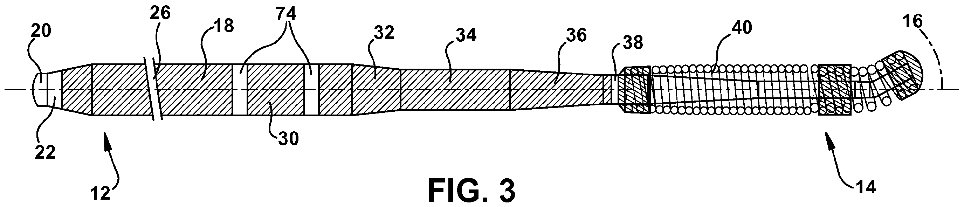

The invention relates to an apparatus 10 in the form of a guidewire for navigating a vessel, such as the human vasculature. The guidewire 10 may be especially suited to traverse a partial or total occlusion of the vessel. The guidewire 10 is illustrated in Figs. 1-4. The guidewire 10 includes a flexible elongated core 20 that has longitudinally spaced proximal and distal ends 12 and 14, respectively. The guidewire 10 also includes a cover 30 that wraps the core 20 and extends along a substantial portion of its length. The guidewire 10 further includes a coil 40 mounted to the core 20 at the distal end 14 of the guidewire.

In this description, the term "longitudinal" is used to refer to a direction defined by the length of the guidewire 10, generally horizontal as viewed in Figs. 1-4 and extending along a central axis 16 of the guidewire 10. The term "lateral" is used herein to refer to a direction which is transverse to the longitudinal direction, *i.e.,* transverse to the guidewire axis 16. The term "radial" is used herein to refer to a direction which is radial with respect to the longitudinal direction, *i.e.,* radial with respect to the guidewire axis 16.

A central body portion 18 of the guidewire 10 extends between the proximal end 12 and distal end 14. The core 20 extends longitudinally from the length of the guidewire 10 from the proximal end 12 to the distal end 14. The core 20 is flexible and may, for example, be constructed of a stainless steel material, such as a grade 304 surgical stainless steel. The core 20 has a proximal end 22 and a distal end 24. The cover 30 wraps the core 20 from the proximal end 12, along the body portion 18, to the distal portion 14. The cover 30 is constructed of a biocompatible material, such as a polymer material. The cover 30 can, for example, be constructed of a PTFE (Polytetrafluoroethylene) resin material. As best shown in Fig. 2, the cover 30 can leave a portion of the distal end 14 of the guidewire 10 uncovered.

The body portion 18 has a generally uniform diameter D1 along its length, which accounts for the majority of the length of the guidewire 10. The diameter D1 can be up to a 0.035 inch (0,889 mm) diameter. For example, the diameter D1 could be a 0.014 inch (0.35mm) diameter. In the embodiment of Figs. 1-4, break lines in the body portion 18, identified generally at 26, are used to indicate a portion of the body portion that is omitted from the figures for purposes of clarity and simplicity. The length of the guidewire 10 can vary depending on its intended application. For instance, the guidewire 10 can be up to 1700-2000mm or longer. In one particular example, the guidewire 10 can have a length of 1800mm.

The guidewire 10 has a diameter that is dictated primarily by the diameter of the core 20. In the embodiment of Figs. 1-4, the guidewire 10 has a generally uniform diameter D1 along its length from the proximal end 12, along the body portion 18 to proximate the distal end 14. For example, the proximal end 12 and body portion 18 may have a nominal diameter of about 0.35mm. The diameter of the guidewire 10 van vary along its length. For example, the distal end 14 of the guidewire 10 may have one or more sections or segments having tapered configurations of varying diameters. The tapered diameters of these segments may, for example, be selected to provide a desired degree of guidewire flexibility.

As shown in Figs. 1-3, the guidewire body 18 has a first tapered segment 32 that tapers down to a first segment 34 with diameter D2, and a second tapered segment 36 that tapers down to a second segment 38 with diameter D3. In the illustrated embodiment, the cover 30 terminates at the second segment 38. The cover 30 could terminate at other positions along the length of the core 20. Referring to Fig. 2, at the distal end 14, the core 20 has a third tapered segment 42 followed by a third segment 44 with diameter D4, and a fourth tapered segment 46 followed by a fourth segment 48 with diameter D5. The fourth segment 48 terminates at the distal tip of the core 20.

Referring to Figs. 1, 3 and 4, the guidewire 10 includes a coil 40 that surrounds the core 20 along the length of the distal end portion 14. A hydrophilic coating may coat or otherwise cover the coil 40. In the illustrated embodiment, the coil 40 surrounds core 20 beginning at the second segment 38 and continues over the third tapered segment 42, third segment 44, fourth tapered segment 46, and fourth segment 48. The extent or coverage of the coil could diverge from that illustrated in the figures. For example, the coil 40 could extend along segments in addition to segments 38, 42, 44, 46, and 48, or one or more of these segments could be left uncovered by the coil.

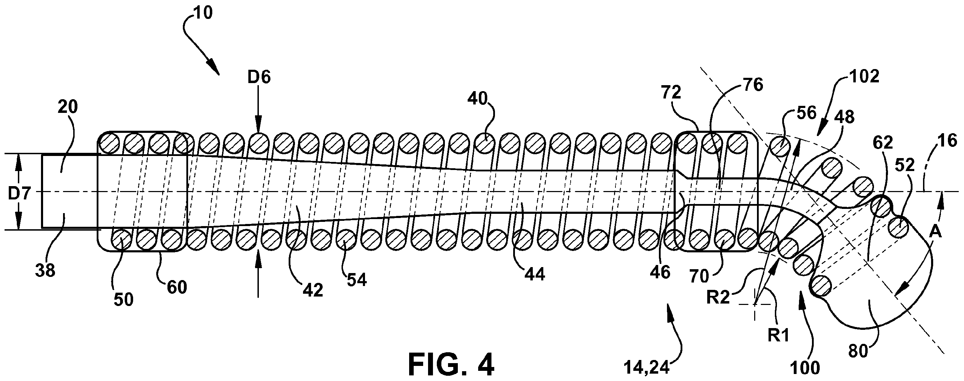

Referring to Fig. 4, the coil 40 has a proximal end 50 and an opposite distal end 52. The coil 40 is interconnected with the core 20 at three locations along the length of the coil. The proximal end 50 of the coil 40 is secured to the second segment 38 of the core 20 by a soldered joint 60 formed, for example, of a gold-tin or silver-tin solder material. The distal end 52 of the coil 40 is secured to the fourth segment 48 at the distal end 24 of the core 20 by a welded joint 62. An interface portion 70 of the coil 40 positioned between the proximal end 50 and distal end 52 is also connected to the core 20 by a soldered joint 72 formed, for example, of a gold-tin or silver-tin solder material. The coil 40 could have a greater number of connections with the core 20 or fewer connections with the core.

The coil has an outer diameter D6 and an inner diameter D7 (*see* Fig. 4). As best shown in Fig. 4, the diameters D6 and D7 can be substantially constant along the length of the coil 40. In the illustrated embodiment, the inner diameter D7 is about equal to the outer diameter D3 of the second segment 38. The diameter of the coil 40 could, however, vary along the length of the coil. For example, the coil diameter could follow the taper of the core 40 at one or both of the tapered sections 42 and 46.

In the embodiment of Figs. 1-4, the coil 40 has a two material construction, with a proximal first coil portion 54 constructed of a stainless steel material, such as a 304 surgical grade stainless steel, and a distal second coil portion 56 constructed of a platinum alloy, such as a platinum-tungsten alloy. The interface portion 70 includes the stainless steel-platinum alloy interface 76, where the first and second coil portions 54 and 56 of the coil 40 meet. The solder joint 72 thus connects both the distal end of the stainless steel first coil portion 54 and the proximal end of the platinum alloy second coil portion 56 to the fourth segment 48 of the core 20. Similarly, the solder joint 60 connects the proximal end of the stainless steel first coil portion 54 to the second segment 38 of the core 20, and the welded joint 62 connects the distal end of the platinum alloy second coil portion 56 to the fourth segment 48 of the core.

The welded joint 62 also defines a distal tip 80 of the guidewire 10. The distal tip 80 has a generally rounded configuration with a diameter about equal to the outer diameter of the coil 40. More specifically, the distal tip 80 has a diameter about equal to the outer diameter of the distal end of the second coil portion 56 of the core 20. Thus, in a construction in which the coil 40 has a multi-diameter configuration, the distal tip 80 may have a diameter that is different than other portions of the coil. Due to its welded construction, the distal tip 80 has a solid, homogeneous material construction that surrounds or encapsulates a distal end portion of the platinum alloy second coil portion 56.

The welded joint 62 is formed by tungsten inert gas (TIG) welding, which can also be referred to as gas tungsten arc welding (GTAW). The welded joint 62 is formed *via* TIG welding using a filler material, such as a surgical grade 304 stainless steel that matches the material of the core 20. The filler material fills the space between the core 20 and the coil 40, encapsulating the coil to thereby form a permanent connection with the coil. Simultaneously, the distal portion 24 of the core 40 may itself become molten or partially molten and combine with the molten filler material to form a homogeneous mixture that encapsulates the coil 40 and thereby forms the permanent connection with the coil.

The second coil portion 56, being constructed of a platinum alloy material, has a melting point that is significantly higher than that of the stainless steel used to form the core 20 and welded joint 62. Thus, the second coil portion 56 resists melting when the TIG/GTAW welding occurs, which allows the welded joint 62 to encapsulate the second coil portion without deforming it. The second coil portion 56 can therefore maintain its helical coil spring configuration and its spring properties throughout and after the formation of the weld joint 62.

The rounded shape of the distal tip 80 can be formed in a variety of manners. For example, the distal tip 80 can be formed in a rough shape during the welding and post-processed, by means such as grinding, polishing, etc. to achieve the final form. Alternatively, the distal portions of the core 20 and coil 40 could be welded and formed simultaneously, such as by welding the distal tip 80 in a mold or other shape-forming confinement constructed of a high-melting point material, such as a ceramic material. The distal tip 80 could, for example, be constructed of a silver brazing alloy or a gold brazing alloy.

The guidewire 10 may also include one or more markers constructed of a radiopaque material, such as gold, platinum, iridium or a combination thereof, such as a platinum-iridium alloy, which can be easily viewed on x-rays. The inclusion of markers facilitates monitoring the progression of the guidewire 10 in a patient's vasculature. The markers can, for example, comprise portions of the core 20 that are plated with these materials. Example locations for markers are illustrated generally at 74 in Fig. 1. Alternative locations could also serve as markers. For example, the tip 80 of the guidewire 10 could be formed or coated with a radiopaque material and thus serve as a marker.

The guidewire 10 has a stiffness that varies along its length due at least in part to factors such as the material construction of the core 20 and the diameter of the core. The term "stiffness" is used herein to indicate the resistance of an elastic body to deflection or deformation by an applied force. With regard to the guidewire 10, its stiffness can be determined, for example, by applying a force axially or longitudinally. The stiffness of the guidewire 10 is judged in terms of the magnitude of the axial/longitudinal force required to cause a predetermined degree of bending.

As shown in Fig. 1, the guidewire 10 can have a substantially uniform construction along a majority of its length. Approaching the distal portion 14, the guidewire, *i.e.,* the core 20 and its accompanying cover 30, are reduced in diameter through the taper portions 32, 36, and 42, and their respective reduced diameter portions 34, 38, and 44. While the term "diameter" is used herein for simplicity, there is no requirement that the guidewire 10 or core 20 have a circular cross-section. Instead, any portion of the guidewire 10 can have any desired cross-sectional shape. In such an instance, the term "diameter" (as used herein) would refer to a cross-sectional dimension commensurate with cross-sectional size or area, as understood by one having ordinary skill in the art. In view of this, it can be appreciated that the guidewire 10 of Figs. 1 and 2 has a stiffness that is reduced over its length as the core 20 tapers down along the distal end 14 toward the distal tip 80.

The coil 40 has properties, *e.g.*, elastic or spring properties, that cause it to resume its helical configuration after being deflected. Additionally, the coil 40 also increases the stiffness of the guidewire 10 along the portions of the core 20 covered by the coil. In the illustrated embodiment, the stiffness of the distal end 14 of the guidewire 10 is essentially equal to the stiffness of the core 20 plus the stiffness of the coil 40. Thus, the overall stiffness of the distal end 14 of the guidewire 10 can be configured to have a predetermined stiffness by selecting the appropriate combination of coil 40 characteristics (*e.g*., materials and configurations) and core 20 characteristics (*e.g*., materials and configurations, diameters, tapers, cross-sections, etc.).

The distal end 14 of the guidewire 10 includes an end segment 100 that extends from the solder joint 72 up to and including the distal tip 80. In the illustrated embodiment, the end segment 100 includes the fourth segment 48 of the core 20, the welded joint 62 forming the distal tip 80, and the platinum alloy second coil portion 56 of the coil 40. The end segment 100 has a bent or curved configuration defined by a bend 102 in which the fourth core segment 48 is bent at an angle relative to the axis 16. The bend angle is indicated generally at A in Fig. 4. The bend angle A can be any desired angle. For example, the bend angle A can be about 24-32 degrees.

Additionally, the bend 102 of the end segment 100 can be configured to occur at any desired location along the length of the distal end 14 of the guidewire 10. For example, the bend 102 can begin at a location that is 0.20mm-1.00mm from the distal tip 80. Other bend locations are possible. To facilitate a desired bend location, the lengths and positions of the tapered segment 46 and core segment 48 can be configured accordingly.

Advantageously, the welded construction of the distal tip 80 permits the bend 102 to be positioned in such close proximity (*e.g*., 0.20mm-1.00mm) to the tip. The weld joint 62, formed from a stainless steel material that matches the material of the core 20, results in a homogeneous construction in which the material properties do not differ substantially at the transition from core 20 to tip 80. Thus, when the bend 102 is applied, the materials of the core and the tip respond similarly or identically and thereby resist any failure (*e.g*., rupture, cracking, *etc*.) that might otherwise occur where different materials are used.

The bend 102 can be produced in the core segment 48 before or after the assembly of the coil 40 to the core 20. Due at least in part to the fact that the bend 102 is defined between the solder joint 72 and the welded joint 62 in which the coil 40 is connected to the core 20, the coil follows the bend. As a result, portions of the coils of the second coil portion 56 are moved close together along an inner bend radius R1 of the bend 102. The inner bend radius R1 can, for example, be 0.10mm-1.70mm. Additionally, portions of the coils of the second coil portion 56 are moved away from each other along an outer radius R2 of the bend 102. The outer bend radius R2 can, for example, be 0.40mm-2.10mm.

This construction of the end segment 100 provides advantageous features. If, during use, a force applied to the end segment 100 causes it to deflect in a manner that unbends or straightens the bend 102, the spaced coil portions along the outer radius R2 can move towards each other to thereby allow the deflection to take place. Once the deflecting force is removed, the bend 102 in the end segment 100 is free to resume its configuration under the resilient properties of the core 20 and those of the coil 40.

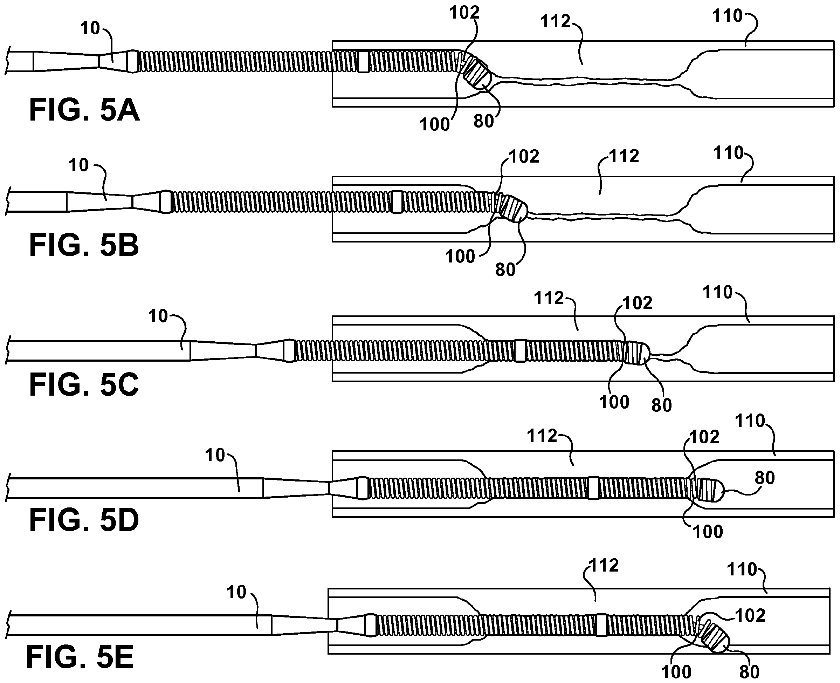

During use, the bend 102 in the end portion 100 facilitates navigating the guidewire 10 in the vasculature to penetrate an occlusion in a lumen. This is illustrated in Figs. 5A-5E. Referring to Fig. 5A, during use, the guidewire 10 approaches an occlusion 112 in a lumen 110 (*i.e.,* a blood vessel). The tip 80 and the bend 102 in the end portion 100 in combination to allow the user/surgeon to navigate the guidewire 10 so that the tip engages the occlusion 112. Navigation can be monitored *via* x-ray using the markers 74 (*see* Figs. 1-3).

Referring to Fig. 5B, the guidewire 10 is navigated and advanced so that the tip 80 enters the occlusion 112. While the guidewire 10 continues advancing in the lumen 110, the end portion 100 deflects, which causes the bend 102 to unbend or straighten. The straightening of the bend 102 allows the guidewire 10 to advance through the occlusion 112, as shown in Fig. 5C. At this point, the bend 102 can be almost completely straightened so that the end portion 100 extends essentially coaxially with the remainder of the guidewire 10.

Referring to Fig. 5D, upon further advancement of the guidewire 10 through the occlusion 112, the tip 80 eventually emerges. As shown in Fig. 5E, after the tip 80 exits the occlusion 112, the end portion 100 emerges and the bend 102 resumes its configuration under the resilience of the second spring portion 56 and the core 10. The bend 102 is therefore available to perform its function in allowing the user/surgeon to navigate the lumen 110, having traversed the occlusion 112.

From the above description of the invention, those skilled in the art will perceive improvements, changes and modifications. Such improvements, changes and modifications within the skill of the art are intended to be covered by the appended claims.

## Claims

1. A coronary guidewire (10), comprising:
a flexible elongated core (20); a coil (40) comprising a plurality of helical coil turns wound around a distal end portion of the core, the coil having a proximal portion (54) constructed of stainless steel and including a proximal end of the coil, and a distal portion (56) constructed of a platinum alloy and including distal end of the coil;
a weld joint (62) that connects the distal end of the coil to the core and defines a distal tip of the guidewire;
a first solder joint (60) that connects the proximal end of the coil to the core; and
a second solder joint (72) that connects the coil to the core at a location where the proximal and distal portions of the coil meet; wherein the distal end portion of the core comprises a flexible bend (102) adjacent the distal tip, the bend beginning at a location that is 0.20-1.00 mm from the distal tip.

2. The guidewire recited in claim 1, wherein the distal tip has a rounded semispherical surface having a diameter about equal to an outside diameter of the coil.

3. The guidewire recited in any of the preceding claims, wherein the distal end portion of the core comprises a bend adjacent the distal tip, the coil following the bend such that adjacent coil turns along the bend are spaced from each other, the spaced coil turns being movable toward each other to permit the bend in the core to straighten.

4. The guidewire recited in any of the preceding claims, wherein the distal end portion of the core comprises a flexible bend adjacent the distal tip, the flexible bend having an inner bend radius of 0.10mm-1.70mm and an outer bend radius of 0.40mm-2.10mm.

5. The guidewire recited in any of the preceding claims, wherein the weld joint comprises a tungsten inert gas welded joint.

6. The guidewire recited in any of the preceding claims, wherein the distal end portion of the core comprises a flexible bend adjacent the distal tip, the flexible bend having a bend angle of 24-32 degrees.

7. The guidewire recited in any of the preceding claims, wherein the first solder joint encompasses substantially three coil turns.

8. The guidewire recited in any of the preceding claims, wherein the weld joint encompasses substantially two coil turns.

9. The guidewire recited in any of the preceding claims, wherein the second solder joint encompasses substantially three coil turns.

10. The guidewire recited in any of the preceding claims, wherein substantially three coil turns are provided between the second solder joint and the weld joint.

## Patentansprüche

1. Koronarer Führungsdraht (10), umfassend:
einen flexiblen, länglichen Kern (20);
eine Wendel (40), umfassend eine Vielzahl von helixförmigen Wendelwindungen, die um einen distalen Endabschnitt des Kerns gewickelt sind, wobei die Wendel einen proximalen Abschnitt (54) aufweist, der aus rostfreiem Stahl hergestellt ist und ein proximales Ende der Wendel aufweist, und einen distalen Abschnitt (56), der aus einer Platinlegierung hergestellt ist und ein distales Ende der Wendel aufweist;
eine Schweißverbindung (62), die das distale Ende der Wendel mit dem Kern verbindet und eine distale Spitze des Führungsdrahtes definiert;
eine erste Lötverbindung (60), die das proximale Ende der Wendel mit dem Kern verbindet; und
eine zweite Lötverbindung (72), welche die Wendel mit dem Kern an einer Stelle verbindet, an der sich die proximalen und distalen Abschnitte der Wendel treffen; wobei der distale Endabschnitt des Kerns eine flexible Biegung (102) angrenzend an die distale Spitze aufweist, wobei die Biegung an einer Stelle beginnt, die 0,20 - 1,00 mm von der distalen Spitze entfernt ist.

2. Führungsdraht nach Anspruch 1, wobei die distale Spitze eine abgerundete, halbkugelförmige Oberfläche mit einem Durchmesser von etwa gleich zu einem Außendurchmesser der Wendel aufweist.

3. Führungsdraht nach einem der vorhergehenden Ansprüche, wobei der distale Endabschnitt des Kerns eine Biegung angrenzend an die distale Spitze umfasst, wobei die Wendel der Biegung so folgt, dass benachbarte Wendelwindungen entlang der Biegung voneinander beabstandet sind, wobei die beabstandeten Wendelwindungen in Richtung zueinander beweglich sind, sodass die Biegung im Kern gerade ausgerichtet werden kann.

4. Führungsdraht nach einem der vorhergehenden Ansprüche, wobei der distale Endabschnitt des Kerns eine flexible Biegung angrenzend an die distale Spitze umfasst, wobei die flexible Biegung einen inneren Biegeradius von
0,10 mm - 1,70 mm und einen äußeren Biegeradius von 0,40 mm - 2,10 mm aufweist.

5. Führungsdraht nach einem der vorhergehenden Ansprüche, wobei die Schweißverbindung eine Wolfram-Inertgas-Schweißverbindung umfasst.

6. Führungsdraht nach einem der vorhergehenden Ansprüche, wobei der distale Endabschnitt des Kerns eine flexible Biegung angrenzend an die distale Spitze umfasst, wobei die flexible Biegung einen Biegewinkel von 24 - 32 Grad aufweist.

7. Führungsdraht nach einem der vorhergehenden Ansprüche, wobei die erste Lötverbindung im Wesentlichen drei Wendelwindungen erfasst.

8. Führungsdraht nach einem der vorhergehenden Ansprüche, wobei die Schweißverbindung im Wesentlichen zwei Wendelwindungen erfasst.

9. Führungsdraht nach einem der vorhergehenden Ansprüche, wobei die zweite Lötverbindung im Wesentlichen drei Wendelwindungen erfasst.

10. Führungsdraht nach einem der vorhergehenden Ansprüche, wobei zwischen der zweiten Lötverbindung und der Schweißverbindung im Wesentlichen drei Wendelwindungen vorgesehen sind.

## Revendications

1. Fil guide coronaire (10) comportant
un noyau flexible allongé (20)
un enroulement en spires (40)
comportant une pluralité de spires hélicoïdales enroulées autour de la partie distale du noyau, l'enroulement ayant une partie proximale (54) en acier inoxydable et ayant une extrémité proximale de l'enroulement et une partie distale (56) en un alliage de platine et ayant à la partie distale de l'enroulement
un joint soudé (62) qui relie l'extrémité distale de l'enroulement au noyau et définit la pointe distale du fil guide
un premier joint de soudure (60) qui relie l'extrémité distale de l'enroulement au noyau et
un second joint de soudure (72) qui relie l'enroulement au noyau à un endroit où les parties proximale et distale de l'enroulement se rejoignent,
dans lequel la partie d'extrémité distale de l'enroulement comporte une courbure flexible (102) adjacente à la pointe distale, la courbure commençant à un endroit situé à 0.20-1.00 mm de la pointe distale.

2. Fil guide selon la revendication 1, dans lequel la pointe distale a une surface semi sphérique arrondie ayant un diamètre presque égal au diamètre externe de l'enroulement.

3. Fil guide selon l'une quelconque des revendications précédentes, dans lequel la partie d'extrémité distale du noyau comporte une courbure adjacente à la pointe distale, l'enroulement suivant la courbure de sorte que les spires de l'enroulement adjacentes à la courbure soient espacées les unes des autres, les spires espacées pouvant se déplacer les unes vers les autres pour permettre à la courbure du noyau de se redresser.

4. Fil guide selon l'une quelconque des revendications précédentes, dans lequel la partie d'extrémité distale du noyau comporte une courbure flexible adjacente à la pointe distale, la courbure flexible ayant un rayon de courbure interne de 0.10mm-1.70mm et un rayon de courbure externe de 0.40mm-2.10mm.

5. Fil guide selon l'une quelconque des revendications précédentes, dans lequel le joint de soudure comporte un joint de soudure au gaz de tungstène inerte.

6. Fil guide selon l'une quelconque des revendications précédentes, dans lequel la partie d'extrémité distale du noyau comporte une courbure flexible adjacente à la pointe distale, la courbure flexible ayant un angle de courbure de 24-32 degrés.

7. Fil guide selon l'une quelconque des revendications précédentes, dans lequel le premier joint de soudure englobe sensiblement trois spires d'enroulement.

8. Fil guide selon l'une quelconque des revendications précédentes, dans lequel le joint de soudure englobe sensiblement deux spires d'enroulement.

9. Fil guide selon l'une quelconque des revendications précédentes, dans lequel le second joint de soudure englobe sensiblement trois spires d'enroulement.

10. Fil guide selon l'une quelconque des revendications précédentes, dans lequel sensiblement trois spires d'enroulement sont disposées entre le second joint de soudure et le joint soudé.
